(19) Europäisches Patentamt
European Patent Office
Office européen des brevets

(11) Veröffentlichungsnummer : **0 086 981**
**B1**

(12) # EUROPÄISCHE PATENTSCHRIFT

(45) Veröffentlichungstag der Patentschrift : **10.09.86**

(21) Anmeldenummer : **83100678.8**

(22) Anmeldetag : **26.01.83**

(51) Int. Cl.⁴ : **C 07 D495/04**, C 07 D519/00, C 07 B 57/00, A 61 K 31/55 // (C07D495/04, 333:00, 243:00),(C07D519/00, 495:00, 451:00)

(54) **Substituierte Thienobenzodiazepinone, Verfahren zu ihrer Herstellung und diese Verbindungen enthaltende Arzneimittel.**

(30) Priorität : **06.02.82 DE 3204169**

(43) Veröffentlichungstag der Anmeldung : **31.08.83 Patentblatt 83/35**

(45) Bekanntmachung des Hinweises auf die Patenterteilung : **10.09.86 Patentblatt 86/37**

(84) Benannte Vertragsstaaten : **AT BE CH DE FR GB IT LI LU NL SE**

(56) Entgegenhaltungen :
**EP-A- 0 039 519**
**US-A- 3 953 430**
**US-A- 4 168 269**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
**D-7950 Biberach (Riss) (DE)**

(72) Erfinder : **Engel, Wolfhard, Dr. Dipl.-Chem.**
**Mozartstrasse 13**
**D-7950 Biberach 1 (DE)**
Erfinder : **Schmidt, Günther, Dr. Dipl.-Chem.**
**Johann-Seb.-Bach-Strasse 27**
**D-7950 Biberach 1 (DE)**
Erfinder : **Eberlein, Wolfgang, Dr. Dipl.-Chem.**
**Obere Au 6**
**D-7950 Biberach 1 (DE)**
Erfinder : **Trummlitz, Günter, Dr. Dipl.-Chem.**
**Buchenweg 27**
**D-7951 Warthausen (DE)**
Erfinder : **Hammer, Rudolf, Dr.**
**Via Fabio Filzi 33**
**Milano (IT)**
Erfinder : **Del Soldato, Piero, Dr.**
**Via E. Toti 22**
**Monza (IT)**

**EP 0 086 981 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue substituierte Thienobenzodiazepinone, Verfahren zu ihrer Herstellung und diese Verbindungen enthaltende Arzneimittel.

In der DE-OS-1 795 176 werden bestimmte Dibenzodiazepinone mit einer ulcushemmenden und sekretionshemmenden Wirkung beschrieben. Aus der US-PS-3 953 430 sind substituierte Dibenzodiazepine mit antidepressiver und analgetischer Wirkung bekannt. In der US-PS-4 168 269 werden substituierte Thienobenzodiazepinone als Ausgangsprodukte für Thienobenzodiazepine mit analgetischer Wirkung beschrieben. Darüber hinaus werden in der EP-A-0 039 519 Thienobenzodiazepinone mit antiulcerogenen und sekretionshemmenden Effekten erwähnt. Es wurden nun Thienobenzodiazepinone mit neuartigen Aminoacylresten gefunden, die gegenüber den oben erwähnten Verbindungen interessante und überlegene pharmakologische Wirkungen aufweisen.

Gegenstand der Erfindung sind :

a) substituierte Thienobenzodiazepinone der allgemeinen Formel I

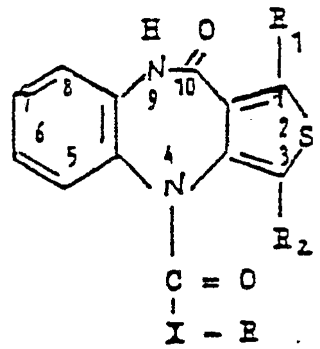

(I)

worin

$R_1$ ein Wasserstoffatom oder einen Alkylrest mit 1 bis 4 Kohlenstoffatomen bedeutet,

$R_2$ ein Halogenatom darstellt oder eine der Bedeutungen von $R_1$ hat,

X das Sauerstoffatom, die —NH- oder —$NCH_3$-Gruppe und

R einen — gegebenenfalls durch eine weitere Methylgruppe substituierten — 1-Methyl-4-piperidinyl-, 4-Methyl-1-piperazinyl- oder einen 3α- oder 3β-Tropanyl-Rest bedeuten,

sowie ihre Säureadditionssalze.

Alkylreste mit 1 bis 4 Kohlenstoffatomen sind der Methyl-, Ethyl-, Propyl-, Isopropyl-, n-Butyl-, Isobutyl-, sek.-Butyl-, tert.-Butylrest.

Ein besonders bevorzugter Alkylrest für $R_1$ und $R_2$ ist der Methylrest. Halogenatome $R_2$ sind das Bromatom, insbesondere das Chloratom. Bevorzugte Verbindungen sind somit Thienobenzodiazepinone der allgemeinen Formel I

worin

$R_1$ ein Wasserstoffatom oder die Methylgruppe,

$R_2$ das Chloratom bedeutet oder eine der Bedeutungen von $R_1$ hat

X das Sauerstofftatom, die NH- oder $NCH_3$-Gruppe und

R einen 1-Methyl-4-piperidinyl-, 4-Methyl-1-piperazinyl-, endo- oder exo-8-Methyl-8-azabicyclo[3,2,1]oct-3-yl-Rest (3α- oder 3β-Tropanylrest) bedeutet.

Die Verbindungen der allgemeinen Formel I können nach Umsetzung mit anorganischen oder organischen Säuren auch in Form ihrer physiologisch verträglichen Salze vorliegen. Als Säuren haben sich hierzu beispielsweise Salzsäure, Bromwasserstoffsäure, Schwefelsäure, Methylschwefelsäure, Phosphorsäure, Weinsäure, Fumarsäure, Zitronensäure, Maleinsäure, Bernsteinsäure, Gluconsäure, Äpfelsäure, p-Toluolsulfonsäure, Methansulfonsäure oder Amidosulfonsäure als geeignet erwiesen.

Besonders bevorzugte Verbindungen der allgemeinen Formel I sind solche, in denen $R_1$ und $R_2$ jeweils ein Wasserstoffatom oder den Methylrest bedeuten, sowie ihre pharmakologisch verträglichen Säureadditionssalze.

Zur Erläuterung des Erfindungsgegenstandes seien als Beispiele folgende Verbindungen genannt :

4,9-Dihydro-4-[[(1-methyl-4-piperidinyl)oxy]carbonyl]-10H-thieno[3,4-b] [1,5]benzodiazepin-10-on,

cis-4,9-Dihydro-4-[[(1,2-dimethyl-4-piperidinyl)oxy]carbonyl]-10H-thieno[3,4-b] [1,5]benzodiazepin-10-on,

trans-4,9-Dihydro-4-[[(1,2-dimethyl-4-piperidinyl)oxy]carbonyl]-10H-thieno[3,4-b] [1,5]benzodiazepin-10-on,

cis-4,9-Dihydro-4-[[(1,3-dimethyl-4-piperidinyl)oxy]carbonyl]-10H-thieno[3,4-b] [1,5]benzodiazepin-10-on,

trans-4,9-Dihydro-4-[[(1,3-dimethyl-4-piperidinyl)oxy]carbonyl]-10H-thieno[3,4-b] [1,5]benzodiazepin-10-on,

4,9-Dihydro-4-[[(1-methyl-4-piperidinyl)amino]carbonyl]-10H-thieno[3,4-b] [1,5] benzodiazepin-10-on,

cis-4,9-Dihydro-4-[[(1,2-dimethyl-4-piperidinyl)amino]carbonyl]-10H-thieno[3,4-b] [1,5]benzodiazepin-10-on,

trans-4,9-Dihydro-4-[[(1,2-dimethyl-4-piperidinyl)amino]carbonyl]-10H-thieno[3,4-b] [1,5]benzodiazepin-10-on,

cis-4,9-Dihydro-4-[[(1,3-dimethyl-4-piperidinyl)amino]carbonyl]-10H-thieno[3,4-b] [1,5]benzodiazepin-10-on,

trans-4,9-Dihydro-4-[[(1,3-dimethyl-4-piperidinyl)amino]-carbonyl]-10H-thieno[3,4-b] [1,5]benzodiazepin-10-on,

4,9-Dihydro-1-methyl-4-[[(1-methyl-4-piperidinyl)oxy]carbonyl]-10H-thieno[3,4-b] [1,5]benzodiazepin-10-on,

4,9-Dihydro-3-methyl-4-[[(1-methyl-4-piperidinyl)oxy]carbonyl]-10H-thieno[3,4-b] [1,5]benzodiazepin-10-on,

4,9-Dihydro-1,3-dimethyl-4-[[(1-methyl-4-piperidinyl)oxy]carbonyl]-10H-thieno[3,4-b] [1,5]benzodiazepin-10-on,

3-Chlor-4,9-dihydro-4-[[(1-methyl-4-piperidinyl)oxy]carbonyl]-10H-thieno[3,4-b] [1,5]benzodiazepin-10-on,

4,9-Dihydro-1-methyl-4-[[(1-methyl-4-piperidinyl)amino]carbonyl]-10H-thieno[3,4-b] [1,5]benzodiazepin-10-on,

4,9-Dihydro-3-methyl-4-[[(1-methyl-4-piperidinyl)amino]carbonyl]-10H-thieno[3,4-b] [1,5]benzodiazepin-10-on,

4,9-Dihydro-1,3-dimethyl-4-[[(1-methyl-4-piperidinyl)amino]-carbonyl]-10H-thieno[3,4-b] [1,5]benzodiazepin-10-on,

3-Chlor-4,9-dihydro-4-[[(1-methyl-4-piperidinyl)amino]carbonyl]-10H-thieno[3,4-b] [1,5]benzodiazepin-10-on,

4,9-Dihydro-4-[[(4-methyl-1-piperazinyl)amino]carbonyl]-10H-thieno[3,4-b] [1,5]benzodiazepin-10-on,

4,9-Dihydro-4-[[(3,4-dimethyl-1-piperazinyl)amino]carbonyl]-10H-thieno[3,4-b] [1,5]benzodiazepin-10-on,

4,9-Dihydro-4-[[(2,4-dimethyl-1-piperazinyl)amino]carbonyl]-10H-thieno[3,4-b] [1,5]benzodiazepin-10-on,

4,9-Dihydro-4-[[(4-methyl-1-piperazinyl)oxy]carbonyl]-10H-thieno[3,4-b] [1,5]benzodiazepin-10-on,

4,9-Dihydro-4-[[(3,4-dimethyl-1-piperazinyl)oxy]carbonyl]-10H-thieno[3,4-b] [1,5]benzodiazepin-10-on,

4,9-Dihydro-4-[[(2,4-dimethyl-1-piperazinyl)oxy]carbonyl]-10H-thieno[3,4-b] [1,5]benzodiazepin-10-on,

4,9-Dihydro-1-methyl-4-[[(4-methyl-1-piperazinyl)amino]carbonyl]-10H-thieno[3,4-b] [1,5]benzodiazepin-10-on,

4,9-Dihydro-3-methyl-4-[[(4-methyl-1-piperazinyl)amino]carbonyl]-10H-thieno[3,4-b] [1,5]benzodiazepin-10-on,

4,9-Dihydro-1,3-dimethyl-4-[[(4-methyl-1-piperazinyl)amino]carbonyl]-10H-thieno[3,4-b] [1,5]benzodiazepin-10-on,

3-Chlor-4,9-dihydro-4-[[(4-methyl-1-piperazinyl)amino]carbonyl]-10H-thieno[3,4-b] [1,5]benzodiazepin-10-on,

4,9-Dihydro-1-methyl-4-[[(4-methyl-1-piperazinyl)oxy]carbonyl]-10H-thieno[3,4-b] [1,5]benzodiazepin-10-on,

4,9-Dihydro-3-methyl-4-[[(4-methyl-1-piperazinyl)oxy]carbonyl]-10H-thieno[3,4-b] [1,5]benzodiazepin-10-on,

4,9-Dihydro-1,3-dimethyl-4-[[(4-methyl-1-piperazinyl)oxy]carbonyl]-10H-thieno[3,4-b] [1,5]benzodiazepin-10-on,

3-Chlor-4,9-dihydro-4-[[(4-methyl-1-piperazinyl)oxy]carbonyl]-10H-thieno[3,4-b] [1,5]benzodiazepin-10-on,

endo-4,9-Dihydro-4-[[(8-methyl-8-azabicyclo[3,2,1]oct-3-yl)oxy]carbonyl]-10H-thieno[3,4-b] [1,5]benzodiazepin-10-on,

exo-4,9-Dihydro-4-[[(8-methyl-8-azabicyclo[3,2,1]oct-3-yl)oxy]carbonyl]-10H-thieno[3,4-b] [1,5]benzodiazepin-10-on,

endo-4,9-Dihydro-4-[[(8-methyl-8-azabicyclo[3,2,1]oct-3-yl)amino]carbonyl]-10H-thieno[3,4-b] [1,5]benzodiazepin-10-on,

exo-4,9-Dihydro-4-[[(8-methyl-8-azabicyclo[3,2,1]oct-3-yl)amino]carbonyl]-10H-thieno[3,4-b] [1,5]benzodiazepin-10-on,

endo-4,9-Dihydro-1-methyl-4-[[(8-methyl-8-azabicyclo[3,2,1]oct-3-yl)oxy]carbonyl]-10H-thieno[3,4-b] [1,5]benzodiazepin-10-on,

endo-4,9-Dihydro-3-methyl-4-[[(8-methyl-8-azabicyclo[3,2,1]oct-3-yl]carbonyl]-10H-thieno[3,4-b] [1,5]-benzodiazepin-10-on,

endo-4,9-Dihydro-1,3-dimethyl-4-[[(8-methyl-8-azabicyclo[3,2,1]oct-3-yl)oxy]carbonyl]-10H-thieno[3,4-b] [1,5]benzodiazepin-10-on,

endo-3-Chlor-4,9-dihydro-4-[[(8-methyl-8-azabicyclo[3,2,1]oct-3-yl)oxy]carbonyl]-10H-thieno[3,4-b] [1,5]benzodiazepin-10-on,

endo-4,9-Dihydro-1-methyl-4-[[(8-methyl-8-azabicyclo[3,2,1]oct-3-yl)amino]carbonyl]-10H-thieno[3,4-b] [1,5]benzodiazepin-10-on,

endo-4,9-Dihydro-3-methyl-4-[[(8-methyl-8-azabicyclo[3,2,1]oct-3-yl)amino] carbonyl]-10H-thieno[3,4-b] [1,5]benzodiazepin-10-on,

endo-4,9-Dihydro-1,3-dimethyl-4-[[(8-methyl-8-azabicyclo[3,2,1]oct-3-yl)amino]carbonyl]-10H-thieno[3,4-b] [1,5]benzodiazepin-10-on,

endo-3-Chlor-4,9-dihydro-4-[[(8-methyl-8-azabicyclo[3,2,1]oct-3-yl)amino]carbonyl]-10H-thieno[3,4-b] [1,5]benzodiazepin-10-on,

exo-4,9-Dihydro-3-methyl-4-[[(8-methyl-8-azabicyclo[3,2,1]oct-3-yl)amino]carbonyl]-10H-thieno[3,4-b] [1,5]benzodiazepin-10-on,

4,9-Dihydro-4-[[N-methyl-N-(1-methyl-4-piperidinyl)amino]carbonyl]-10H-thieno[3,4-b] [1,5]benzodiazepin-10-on,

4,9-Dihydro-1-methyl-4-[[N-methyl-N-(1-methyl-4-piperidinyl)amino]carbonyl]-10H-thieno[3,4-b] [1,5]benzodiazepin-10-on,

4,9-Dihydro-3-methyl-4-[[N-methyl-N-(1-methyl-4-piperidinyl)amino]carbonyl]-10H-thieno[3,4-b] [1,5]benzodiazepin-10-on,

4,9-Dihydro-1,3-dimethyl-4-[[N-methyl-N-(1-methyl-4-piperidinyl)amino]carbonyl]-10H-thieno[3,4-b] [1,5]benzodiazepin-10-on,

3-Chlor-4,9-dihydro-4-[[N-methyl-N-(1-methyl-4-piperidinyl)amino]carbonyl]-10H-thieno[3,4-b][1,5]benzodiazepin-10-on.

Gegenstand der Erfindung sind darüber hinaus :

b) neuartige substituierte Thienobenzodiazepinone der allgemeinen Formel Ia

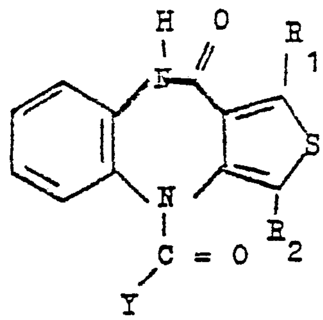

(Ia)

worin $R_1$ und $R_2$ die oben angegebenen Bedeutungen haben und Y ein Halogenatom, bevorzugt das Brom- oder Chloratom, oder den Rest $OR_3$ bedeutet, wobei $R_3$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt.

$R_3$ kann beispielsweise die Bedeutung eines Methyl-, Ethyl-, n-Butyl-, Isobutyl-, Benzyl-, 9-Fluorenylmethyl-, Phenyl-, 4-Nitrophenyl-, 2, 2, 2-Trichlorethyl-, 2,4,5-Trichlorphenyl- oder 2,2,2-Trichlor-tert.butyl-Restes annehmen.

Die substituierten Thienobenzodiazepinone der allgemeinen Formel Ia sind wertvolle Zwischenprodukte bei der Herstellung der pharmakologisch wirksamen und therapeutisch einsetzbaren erfindungsgemäßen Verbindungen der allgemeinen Formel I.

Ein weiterer Gegenstand der Erfindung sind :

c) Arzneimittel, die ein oder mehrere Thienobenzodiazepinone der allgemeinen Formel I enthalten.

Die Verbindungen der allgemeinen Formel I lassen sich hierzu in an sich bekannter Weise in die üblichen pharmazeutischen Zubereitungsformen, z. B. in Lösungen, Suppositorien, Tabletten, Dragees, Kapseln oder Teezubereitungen einarbeiten. Die Tagesdosis bei oraler Gabe liegt im allgemeinen zwischen 0,01 und 5, vorzugsweise 0,02 und 2,5, insbesondere 0,05 und 1,0 mg/kg Körpergewicht, die gegebenenfalls in Form mehrerer, vorzugsweise 1 bis 3 Einzelgaben zur Erzielung der gewünschten Ergebnisse verabreicht wird.

Die substituierten Thienobenzodiazepinone der allgemeinen Formel I und ihre Säureadditionssalze besitzen wertvolle Eigenschaften, die sie gewerblich verwertbar machen, insbesondere sind sie durch eine ausgezeichnete Magen- und Darmschutzwirkung bei Warmblütern gekennzeichnet ; sie hemmen z. B. die Bildung von Magengeschwüren. Ferner weisen sie, bedingt durch eine geringe Toxizität und das Fehlen wesentlicher Nebenwirkungen eine günstige therapeutische Breite auf.

Die ausgezeichnete Wirksamkeit der substituierten Thienobenzodiazepinone der allgemeinen Formel I und ihrer pharmakologisch d. h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden können. Beispielsweise können mit ihnen akute und chronische Ulcera ventriculi und duodeni, Gastritis und hyperacider Reizmagen bei Mensch und Tier behandelt werden.

Sollen die erfindungsgemäßen substituierten Thienobenzodiazepinone der allgemeinen Formel I und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat ; Sekretionshemmer, wie $H_2$-Blocker, z. B. Cimetidin, Ranitidin ; Magen- und Darmtherapeutika, z. B. Metoclopramid, Bromoprid, Tiaprid ; Tranquilizer, wie Benzodiazepine, beispielsweise Diazepam, Oxazepam ; Spasmolytika, z. B. Bietamiverin, Camylofin ; Anticholinergica, z. B. Oxyphencyclimin, Phencarbamid ; Glucocorticoide, wie Prednisolon, Fluocortolon, Betamethason ; nichtsteroidale Antiphlogistika, wie Arylessigsäuren und -propionsäuren, Heteroarylessigsäuren und -propionsäuren, Benzothiazincarboxamiddioxide, Pyrazolidindione, Chinazolinone, z. B. Ibuprofen, Naproxen, Diclofenac, Fenbufen, Flurbiprofen, Indometacin, Lonazolac, Sudoxicam, Piroxicam, Phenylbutazon, Bumadizon-Calcium, Proquazon ; Lokalanaesthetika, beispielsweise Tetracain, Procain ; gegebenenfalls auch Fermente, Vitamine, Aminosäuren etc. enthalten.

d) Ein weiterer Gegenstand der Erfindung sind Verfahren zur Herstellung der substituierten Thienobenzodiazepinone der allgemeinen Formel I

(Siehe Figur Seite 6 f.)

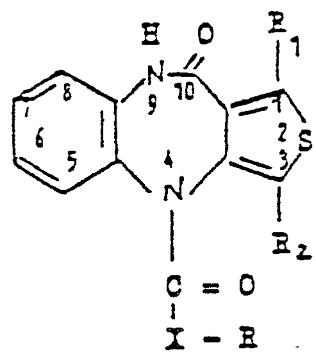

(I)

worin

$R_1$ ein Wasserstoffatom oder einen Alkylrest mit 1 bis 4 Kohlenstoffatomen bedeutet,

$R_2$ ein Halogenatom darstellt oder eine der Bedeutungen von $R_1$ hat,

X das Sauerstoffatom, die NH- oder $NCH_3$-Gruppe und

R einen — gegebenenfalls durch eine weitere Methylgruppe substituierten — 1-Methyl-4-piperidinyl-, 4-Methyl-1-piperazinyl-, endo- oder exo-8-Methyl-8-azabicyclo-[3,2,1]oct-3-yl-Rest bedeutet, und ihrer Säureadditionssalze, sowie zur Herstellung der als Zwischenprodukte verwendeten Thienobenzodiazepinone der allgemeinen Formel Ia

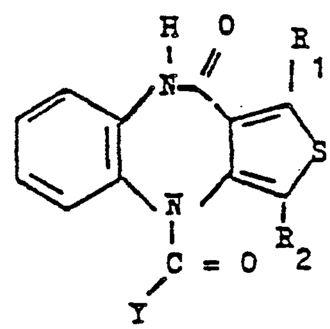

(Ia)

worin

$R_1$ und $R_2$ die oben angegebenen Bedeutungen haben und Y ein Halogenatom, bevorzugt das Brom- oder Chloratom oder den Rest $OR_3$ bedeutet, wobei

$R_3$ einen gegebenenfalls halogensubstituierten Alkylrest mit 1 bis 3 Kohlenstoffatomen, einen gegebenenfalls durch Halogen oder Nitro substituierten Phenylrest oder einen Aralkylrest mit 7 bis 15 Kohlenstoffatomen, beispielsweise einen Methyl-, Ethyl-, n-Butyl-, Isobutyl-, Benzyl-, 9-Fluorenylmethyl-, Phenyl-, 4-Nitrophenyl-, 2,2,2-Trichlorethyl-, 2,4,5-Trichlorphenyl- oder 2,2,2-Trichlor-tert. butyl-Rest darstellt.

Die Verfahren zur Herstellung von Verbindungen der allgemeinen Formel I sind dadurch gekennzeichnet, daß man

a) Thienobenzodiazepinone der allgemeinen Formel Ia nach an sich bekannten Methoden mit Verbindungen der allgemeinen Formel II

$$H—X—R \qquad (II)$$

in der X und R die eingangs angegebenen Bedeutungen haben, zur Reaktion bringt. Die Umsetzung wird ohne oder bevorzugt in Gegenwart inerter Lösungsmittel, z. B. von Wasser, Toluol oder von Alkoholen, wie z. B. Methanol, Ethanol oder Isopropanol, ganz bevorzugt jedoch in Gegenwart aprotischer polarer Lösungsmittel, z. B. von Tetrahydrofuran, 1,4-Dioxan, Acetonitril, N,N-Dimethylformamid, Dimethylsulfoxid, Hexamethylphosphorsäuretriamid, oder von Gemischen davon und bei Temperaturen zwischen 0 °C und dem Siedepunkt des betreffenden Lösungsmittels, bevorzugt zwischen 40 und 100 °C durchgeführt. Bewährt hat sich die Verwendung zusätzlicher anorganischer oder organischer Basen, z. B. von Alkali- oder Erdalkalihydroxiden, -alkoholaten oder -carbonaten, etwa von Natriumhydroxid, Natriummethanolat, Kalium-tert.butanolat, Natriumcarbonat, Kaliumcarbonat ; von tertiären Aminen, z. B. Triethylamin, Ethyldiisopropylamin, N,N-Dimethylanilin oder von Pyridin ; sowie die Umsetzung in Gegenwart eines Überschusses einer Verbindung der allgemeinen Formel II.

b) Gute Ergebnisse erzielt man auch durch Umsetzung eines Thienobenzodiazepinons der allgemeinen Formel Ia mit einer Metallverbindung der allgemeinen Formel IIa

$$M—X—R \qquad (IIa)$$

in der M ein Alkalimetallatom oder 1 Äquivalent eines Erdalkalimetallatoms bedeutet. Dabei lassen sich Metallverbindungen der allgemeinen Formel IIa aus II durch Umsetzung mit Alkali- oder Erdalkalimetallen, etwa mit Natrium, Kalium oder Barium, oder mit Alkali- oder Erdalkalihydriden, etwa mit Natrium-, Kalium- oder Calciumhydrid, oder durch Reaktion mit Alkali- oder Erdalkaliorganylen, z. B. mit n-Butyllithium oder Phenyllithium, *in situ* leicht herstellen.

c) Die Verbindungen der allgemeinen Formel I lassen sich auch durch Umsetzung eines Thienobenzodiazepinons der allgemeinen Formel III

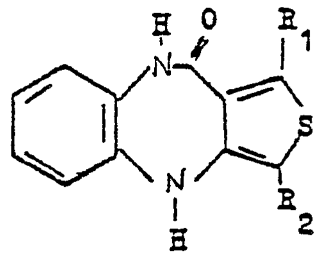

(III)

worin $R_1$ und $R_2$ wie oben angegeben definiert sind, mit Chlorkohlensäurederivaten der allgemeinen Formel V

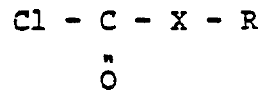

(V)

oder mit Isocyanaten der allgemeinen Formel Va

$$O = C = N—R \qquad \text{(Va)}$$

worin X und R die oben angegebenen Bedeutungen besitzen, herstellen. Die Umsetzung wird in inerten organischen Lösungsmitteln, beispielsweise in aromatischen Kohlenwasserstoffen, wie Toluol, Chlorbenzol oder Xylol, in Ethern, wie Diisopropylether, Tetrahydrofuran oder Dioxan, in offenkettigen oder cyclischen aliphatischen Ketonen, wie 3-Pentanon, in chlorierten aliphatischen Kohlenwasserstoffen, wie 1,2-Dichlorethan, oder in anderen Lösungsmitteln, wie Acetonitril oder Dimethylformamid, oder in Gemischen davon, gegebenenfalls in Gegenwart tertiärer organischer Basen, wie Pyridin, und bei Temperaturen bis zum Siedepunkt des Reaktionsgemisches durchgeführt.

So erhaltene Basen der allgemeinen Formel I können anschließend in ihre Säureadditionssalze oder erhaltene Säureadditionssalze in die freien Basen oder andere pharmakologisch verträgliche Säureadditionssalze übergeführt werden.

Ein Teil der erfindungsgemäßen Thienobenzodiazepinone der allgemeinen Formel I enthält ein oder zwei asymmetrische Kohlenstoffatome in der Seitenkette —CO—X—R. Diese Verbindungen können deshalb in zwei diasteromeren cis- und trans-Formen oder jeweils als enantiomere (+)- und (—)-Formen auftreten. Die Erfindung umfaßt die einzelnen Isomeren ebenso wie ihre Gemische.

Die Trennung der jeweiligen Diastereomeren gelingt auf Grund der unterschiedlichen physikochemischen Eigenschaften, z. B. durch fraktioniertes Umkristallisieren aus geeigneten Lösungsmitteln.

Die Spaltung evtl. Razemate der Verbindungen der allgemeinen Formel I kann nach bekannten Verfahren durchgeführt werden, beispielsweise unter Verwendung einer optisch aktiven Säure, wie (+)- oder (—)-Weinsäure, oder eines Derivats davon, wie (+)- oder (—)-Diacetylweinsäure, (+)- oder (—)-Monomethyltartrat oder (+)-Camphersulfonsäure.

Nach einem üblichen Verfahren zur Isomerentrennung wird das Razemat einer Verbindung der allgemeinen Formel I mit einer der vorstehend angegebenen optisch aktiven Säuren in äquimolarer Menge in einem Lösungsmittel umgesetzt und die erhaltenen kristallinen optisch aktiven Salze werden unter Ausnutzung ihrer verschiedenen Löslichkeit getrennt. Diese Umsetzung kann in jeder Art von Lösungsmittel durchgeführt werden, solange dieses einen ausreichenden Unterschied in der Löslichkeit der Salze aufweist. Vorzugsweise werden Methanol, Ethanol oder deren Gemisch, beispielsweise im Volumenverhältnis 50 : 50, verwendet. Sodann wird jedes der optisch aktiven Salze in Wasser gelöst, mit einer Base, wie Natriumcarbonat oder Kaliumcarbonat, neutralisiert und dadurch die entsprechende freie Verbindung in der (+)- oder (—)-Form erhalten.

Die erfindungsgemäßen Verbindungen der allgemeinen Formel Ia erhält man durch Umsetzung eines Thienobenzodiazepinons der allgemeinen Formel III

(Siehe Figur Seite 8 f.)

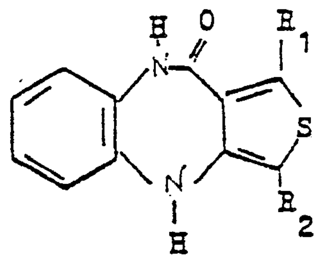

(III)

worin $R_1$ und $R_2$ die eingangs angegebene Bedeutung haben, mit Halogenkohlensäurederivaten der allgemeinen Formel IV

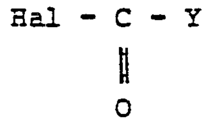

. (IV)

in der Hal das Brom-, bevorzugt das Chloratom bedeutet und Y die oben angegebenen Bedeutungen hat. Die Reaktion wird in inerten organischen Lösungsmitteln, beispielsweise aromatischen Kohlenwasserstoffen, wie Toluol, Chlorbenzol oder Xylol ; offenkettigen oder cyclischen Ethern, wie Diisopropylether, Tetrahydrofuran oder Dioxan ; offenkettigen oder cyclischen aliphatischen Ketonen, beispielsweise 3-Pentanon ; chlorierten aliphatischen Kohlenwasserstoffen, wie 1,2-Dichlorethan oder anderen Lösungsmitteln, wie Acetonitril oder Dimethylformamid oder in Gemischen davon und in Gegenwart tertiärer organischer Basen, bevorzugt von Pyridin, und bei Temperaturen bis höchstens zum Siedepunkt des verwendeten Lösungsmittels oder Lösungsmittelgemisches, bevorzugt zwischen + 30 und + 100 °C, durchgeführt.

Säureadditionssalze erhält man durch Auflösen der erhaltenen freien Base in einem geeigneten Lösungsmittel, z. B. Wasser, Aceton, einem Alkanol, wie Ethanol oder Isopropanol, einem offenkettigen oder cyclischen Ether, wie Diethylether oder Tetrahydrofuran, das die gewünschte Säure enthält oder dem die gewünschte Säure anschließend zugegeben wird. Die Salze werden durch Filtrieren, Ausfällen mit einem Nichtlösungsmittel für das Säureadditionssalz oder durch Verdampfen des Lösungsmittels gewonnen. Salze können auch durch Überführung in die Base und weitere Umsetzung mit einer anderen Säure in andere Salze, z. B. pharmakologisch verträgliche Säureadditionssalze, übergeführt werden.

Die Ausgangsverbindungen der allgemeinen Formel II sind bekannt oder können in Analogie zu literaturbekannten Verfahren bereitet werden. Beispielsweise erhält man 1-Hydroxy-4-methylpiperazin nach S.M. Riba, A.S. Issa und Y.A. Beltagy, Pharmazie *33*, 711 (1978) durch Umsetzung von Bis[N-(2-Chlorethyl)]methylamin mit Hydroxylaminhydrochlorid in wäßrigethanolischer Lösung und in Gegenwart von Kaliumcarbonat.

Die Herstellung der Ausgangsverbindungen der allgemeinen Formel III erfolgt gemäß der US-PS 3 953 430 durch Umsetzung von o-Phenylendiamin mit einem Tetrahydrothiophencarbonsäurederivat der allgemeinen Formel VI

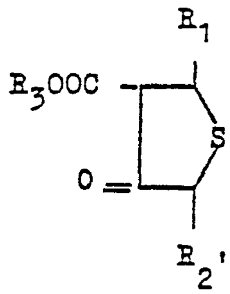

(VI)

in der $R_1$ wie oben definiert ist, $R_2'$ ein Wasserstoffatom oder eine Alkylgruppe mit 1 bis 4 Kohlenstoffatomen und $R_3$ ein Wasserstoffatom oder eine Alkylgruppe mit 1 bis 5 Kohlenstoffatomen bedeuten. Die Umsetzung erfolgt beispielsweise in einem inerten Lösungsmittel, wie Toluol, bei Temperaturen bis zum Siedepunkt des Reaktionsgemisches. Es entsteht dabei ein Tetrahydrothienobenzodiazepinon der allgemeinen Formel VII

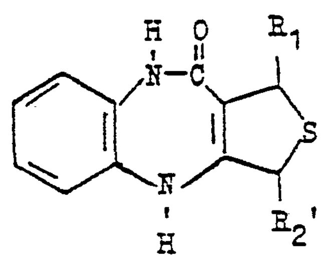

(VII)

welches anschließend mit einem Dehydrierungsmittel, z. B. N-Bromsuccinimid in Dimethylformamid, zu den entsprechenden Verbindungen der allgemeinen Formel III, worin $R_2$ die Bedeutung von $R_2'$ besitzt, umgesetzt wird. Soll daraus eine Verbindung der allgemeinen Formel III hergestellt werden, in der $R_2$ die Bedeutung eines Halogenatoms besitzt, so wird eine Verbindung der allgemeinen Formel III, in der $R_2$ die Bedeutung eines Wasserstoffatoms hat, halogeniert nach an sich üblichen Methoden.

So werden beispielsweise erhalten :

3-Chlor-4,9-dihydro-10H-thieno[3,4-b] [1,5]benzodiazepin-10-on,

4,9-Dihydro-3-methyl-10H-thieno[3,4-b] [1,5]benzodiazepin-10-on, F. 228-230 °C (Methanol),

4,9-Dihydro-1,3-dimethyl-10H-thieno[3,4-b] [1,5]benzodiazepin-10-on, F. 195-196 °C,

4,9-Dihydro-1-methyl-10H-thieno[3,4-b] [1,5]benzodiazepin-10-on, F. 274-276 °C (n-Propanol).

Halogenkohlensäurederivate der allgemeinen Formel IV sind bekannt.

Chlorkohlensäurederivate der allgemeinen Formel V und Isocyanate der allgemeinen Formel Va sind bekannt oder können in Analogie zu literaturbekannten Verfahren erhalten werden (I. W. Mathison u. a., J. Pharm. Sci. *62*, 158 [1963] ; H. Hopff und H. Ohlinger, Angew. Chem. *61*, 183 [1949] : W. Siefken, Liebigs Ann. Chem. *562*, 75 [1949] ; Houben-Weyl VIII, 117 ; Ullmann V, 72 ; L. C. Raiford und K. Alexander, J. Org. Chem. *5*, 306 [1940] ; H. H. Saunders und R. J. Slocombe, Chem. Rev. *43*, 203 [1948] ; R. J. Slocombe, E. E. Hardy, J. H. Saunders und R. L. Jenkins, J. Amer. chem. Soc. *72*, 1888 [1950] ; H. Habad und A. G. Zeiler, Chem. Rev. *73*, 75 [1973]).

Wie bereits oben erwähnt, weisen die neuen Verbindungen der allgemeinen Formel I wertvolle pharmakologische Eigenschaften auf ; insbesondere besitzen sie antiulcerogene und magensäuresekretionshemmende Wirkungen, sowie günstige Effekte auf verschiedene andere Erkrankungen des Magen-Darm-Traktes, unter denen das Colon irritabile besonders hervorgehoben sei.

Eine günstige Relation zwischen antiulcerogenen und antisekretorischen Wirkungen einerseits und den vor allem bei Therapeutika mit anticholinerger Wirkkomponente auftretenden unerwünschten Wirkungen auf die Pupillenweite und Tränen- und Speichelsekretion andererseits ist für die therapeutische Verwendung der Substanzen von besonderer Wichtigkeit. Die folgenden Versuche zeigen, daß die erfindungsgemäßen Verbindungen diesbezüglich überraschend günstige Relationen aufweisen.

Untersuchung auf Selektivität der antimuscarinischen Wirkung

Ziel :

Oxotremorin, ein spezifischer Agonist an muscarinischen Rezeptoren, erzeugt bei Ratten Läsionen der Magenschleimhaut und steigert die Speichelsekretion. Dieses Versuchsmodell wurde gewählt, um eine selektive Wirkung einer antimuscarinischen Substanz auf den Magen erkennen zu können.

Methodik :

Verwendet wurden 10 weibliche Albino-Ratten [Stamm Crl : COBS-CD (SD) BR] pro Behandlungsgruppe mit einem Körpergewicht von 120 bis 150 g, die bei freiem Zugang zu Trinkwasser 24 Stunden vor Versuchsbeginn ohne Nahrung waren.

Um in Vorversuchen die muscarinische Wirkung von Oxotremorin auf jedes der untersuchten Symptome zu ermitteln, wurde mit jeweils mindestens drei Dosen für jedes Symptom eine Dosis-Wirkungskurve erstellt.

Bei der Prüfung antimuscarinischer Substanzen wurde die Oxotremorin-Dosis gewählt, die in den Vorversuchen das zu beeinflussende Symptom bei 90 bis 100 % der Tiere ausgelöst hatte.

| | |
|---|---|
| Magenschleimhaut-Läsionen : | 0,62 mg/kg i. v. |
| Speichelsekretion : | 0,083 mg/kg i. v. |

Jede antimuscarinische Substanz wurde in gleichmäßig abgestuften Dosierungen 15 Minuten vor der Oxotremorin-Gabe intravenös verabreicht. Kontrollgruppen erhielten an Stelle der Prüfsubstanz das Lösungs- und Suspensionsmittel in entsprechender Menge.

Unmittelbar nach Oxotremorin-Gabe wurden die Tiere in einem Glaskäfig für 15 Minuten beobachtet.

Die Prüfung auf Beeinflussung der Oxotremorin-induzierten Speichelsekretion wurde blind durchgeführt, d. h. der Untersucher wußte nicht, wie die Tiere vorbehandelt waren.

Die Ergebnisse wurden als prozentuale Hemmung des Oxotremorin-Effektes (Prozentsatz der Tiere ohne das entsprechende Symptom) ausgedrückt. $ED_{50}$-Werte wurden nach der Methode von LITCHFIELD und WILCOXON (J. Pharmacol. Exp. Ther. *96*, 99, 1949) ermittelt.

Die Effekte auf Schleimhaut-Läsionen des Magens wurden folgendermaßen bewertet :

Die Magenschleimhaut-Läsionen wurden erzeugt durch intravenöse Injektion von 0,62 mg/kg Oxotremorin 30 Minuten nach oraler Gabe von 1 mg/kg Neostigmin (Cholinesterase-Hemmer). 60 Minuten nach Neostigmin-Gabe wurden die Tiere getötet, die Mägen entnommen, geöffnet und auf Vorhandensein von Schleimhaut-Läsionen geprüft. Die schützende Wirkung der Prüfsubstanzen wurde als prozentuale Hemmung (Prozentsatz der Tiere ohne Läsionen) ausgedrückt. $ED_{50}$- bzw. $ED_{70}$-Werte

wurden nach der Methode von LITCHFIELD und WILCOXON (s. o.) ermittelt.

Mydriasis

Der Effekt von Testsubstanzen auf die Pupillenweite von Ratten wurde folgendermaßen untersucht : Die Substanzen wurden an Gruppen von jeweils 10 Tieren in wenigstens 3 gleichmäßig abgestuften Dosierungen intravenös appliziert. Die Pupillenweite wurde anschließend 10 Minuten lang auf evtl. Veränderungen (Auftreten von Mydriasis oder Miosis) beobachtet, und zwar wiederum blind, d. h. dem jeweiligen Untersucher war die Vorbehandlung der Tiere unbekannt. Es wurde der Prozentsatz der Versuchstiere ermittelt, bei denen eine Mydriasis auftrat. $ED_{50}$-Werte wurden wiederum nach LITCHFIELD und WILCOXON (s. o) bestimmt.

2. Bindungsstudien an muscarinischen Rezeptoren :
Bestimmung des $IC_{50}$-Wertes

Als Organspender dienten männliche Sprague-Dawley-Ratten mit 180-220 g Körpergewicht. Nach Entnahme von Herz, Magen und Großhirnrinde wurden alle weiteren Schritte im eiskalten Hepes-HCl-Puffer (pH 7,4 ; 100 m molar NaCl, 10 m molar $MgCl_2$) vorgenommen. Die glatte Muskulatur des Magenfundus wurde von der Magenschleimhaut freipräpariert und vorhomogenisiert. Das Gesamtherz wurde mit einer Schere zerkleinert. Alle Organe wurden abschließend in einem Potter homogenisiert.
Für den Bindungstest wurden die Organhomogenate in folgender Weise verdünnt :

| | |
|---|---|
| Glatter Muskel Magenfundus | 1 : 100 |
| Gesamtherz | 1 : 250 |
| Großhirnrinde | 1 : 3000 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30 °C. Die Inkubationsdauer betrug 45 Minuten. Als Radioligand wurde 0,3 n molar $^3$H-N-Methylscopolamin ($^3$H-NMS) verwendet. Nach Beendigung der Inkubation durch Zentrifugation bei 14 000 g wurde die Radioaktivität im Pellet bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 $\mu$ molar Chinuclidinylbenzilat gebunden wurde. Es wurden immer Vierfachbestimmungen vorgenommen. Die $IC_{50}$-Werte der nichtmarkierten Testsubstanzen wurden graphisch bestimmt. Sie repräsentieren jene Konzentration der Testsubstanz, bei welcher die spezifische Bindung von $^3$H-NMS an die muscarinischen Rezeptoren in den verschiedenen Organen um 50 % gehemmt wurde.
Nach den vorstehenden Angaben wurden beispielsweise die folgenden Verbindungen untersucht :

A = 4,9-Dihydro-4-[[(1-methyl-4-piperidinyl)oxy] carbonyl]-10H-thieno [3,4-b] [1,5] benzodiazepin-10-on

B = 4,9-Dihydro-4-[[(1-methyl-4-piperidinyl)amino] carbonyl]-10H-thieno [3,4-b] [1,5] benzodiazepin-10-on

C = 4,9-Dihydro-4-[[(4-methyl-1-piperazinyl) amino] carbonyl]-10H-thieno [3,4-b] [1,5] benzodiazepin-10-on

(Siehe Tabelle Seite 11 f.)

Ergebnisse

| Substanz | Rezeptor-Bindungs-Tests $IC_{50}$ [n Mol $l^{-1}$] Cortex | Glatter Muskel Magenfundus | Herz | Oxotremorin-Tests [µg/kg] i.v. Antiulcerogene Wirkung $ED_{50}$ | $ED_{70}$ | Salivations-hemmung $ED_{50}$ | Mydriasis $ED_{50}$ [µg/kg] i.v. |
|---|---|---|---|---|---|---|---|
| A | 40 | 200 | 180 | 17 | 25 | 100 | 110 |
| B | 30 | 300 | 220 | 17 | 44 | 40 | 37 |
| C | 20 | 200 | 90 | | 19 | | 30 |

Die Angaben in der vorstehenden Tabelle beweisen, daß die genannten Verbindungen generell eine hohe Affinität zu muscarinischen Rezeptoren besitzen. Darüber hinaus kann man den Daten entnehmen, daß die neuen Verbindungen der allgemeinen Formel I zwischen muscarinischen Rezeptoren verschiedener Gewebe unterscheiden. Dies folgt aus den beträchtlich niedrigeren $IC_{50}$-Werten bei Untersuchung an Präparaten aus der Großhirnrinde gegenüber solchen aus der glatten Muskulatur von Magen und Herz.

Aus den pharmakologischen Daten der vorstehenden Tabelle ergibt sich — in völliger Übereinstimmung mit den Rezeptor-Bindungs-Studien-, daß die Entstehung Oxotremorin-induzierter Magenschleimhaut-Läsionen durch die genannten Verbindungen bereits bei Dosierungen gehemmt wird, bei denen noch keine Einschränkung der Speichelsekretion und keine Mydriasis beobachtet wird.

Die folgenden Beispiele dienen zur näheren Erläuterung der Erfindung. « F » bedeutet « Schmelzpunkt », « Z » bedeutet « Zersetzung ».

Beispiel 1

4-Chlorcarbonyl-4,9-dihydro-10H-thieno [3,4-b] [1,5] benzodiazepin-10-on

16,2 g (0,075 Mol) 4,9-Dihydro-10H-thieno [3,4-b] [1,5] benzodiazepin-10-on wurden in einem Gemisch von 160 ml Diethylketon und 5,9 g (0,075 Mol) Pyridin bei 40 °C innerhalb von 20 Minuten mit 75 ml einer 20-prozentigen Lösung von Phosgen (0,15 Mol) in Toluol versetzt. Man rührte das Reaktionsgemisch 2 Stunden bei 40 °C und anschließend 3 Stunden bei 60 °C. Nach dem Abkühlen auf Raumtemperatur wurde mit 150 ml Wasser verrührt, filtriert und die organische Phase abgetrennt. Diese wurde im Vakuum eingedampft, der Rückstand wurde aus Acetonitril umkristallisiert. Man erhielt das 4-Chlorcarbonyl-4,9-dihydro-10H-thieno [3,4-b] [1,5] benzodiazepin-10-on vom F. 244-245 °C (Z.) in einer Ausbeute von 50 % der Theorie.

Entsprechend erhielt man :

aus 4,9-Dihydro-10H-thieno [3,4-b] [1,5] benzodiazepin-10-on und Chlorkohlensäuremethylester in Dioxan/Toluol-Gemisch das 4,9-Dihydro-4-methoxycarbonyl-10H-thieno [3,4-b] [1,5] benzodiazepin-10-on,

aus 4,9-Dihydro-1-methyl-10H-thieno [3,4-b] [1,5] benzodiazepin-10-on und Phosgen in Dioxan/Toluol-Gemisch das 4-Chlorcarbonyl-4,9-dihydro-1-methyl-10H-thieno [3,4-b] [1,5]-benzodiazepin-10-on vom F. 235-236 °C (aus Chloroform) ; aus 4,9-Dihydro-3-methyl-10H-thieno [3,4-b] [1,5] benzodiazepin-10-on und Phosgen in Dioxan/Toluol-Gemisch das 4-Chlorcarbonyl-4,9-dihydro-3-methyl-10H-thieno [3,4-b] [1,5]-benzodiazepin-10-on ;

aus 4,9-Dihydro-1,3-dimethyl-10H-thieno [3,4-b] [1,5] benzodiazepin-10-on und Phosgen in Dioxan/Toluol-Gemisch das 4-Chlorcarbonyl-4,9-dihydro-1,3-dimethyl-10H-thieno [3,4-b] [1,5]-benzodiazepin-10-on als amorphes schaumiges Produkt, das ohne Reinigung weiter umgesetzt wurde ;

aus 3-Chlor-4,9-dihydro-10H-thieno [3,4-b] [1,5] benzodiazepin-10-on und Phosgen in Diethylketon das 3-Chlor-4-chlorcarbonyl-4,9-dihydro-10H-thieno [3,4-b] [1,5] benzodiazepin-10-on vom F. 238-240 °C (Ethanol) ; aus 4,9-Dihydro-10H-thieno [3,4-b] [1,5] benzodiazepin-10-on und Chlorkohlensäurebenzylester in Dioxan/Toluol-Gemisch das 4-Benzyloxycarbonyl-4,9-dihydro-10H-thieno[3,4-b] [1,5] benzodiazepin-10-on.

Beispiel 2

4,9-Dihydro-4-[[(1-methyl-4-piperidinyl) amino] carbonyl]-10H-thieno [3,4-b] [1,5] benzodiazepin-10-on

Eine Suspension von 3,0 g (0,010 8 Mol) 4-Chlorcarbonyl-4,9-dihydro-10H-thieno [3,4-b] [1,5] benzodiazepin-10-on und 1,2 g (0,010 8 Mol) Natriumcarbonat in 100 ml Acetonitril wurde bis zum Rückfluß erhitzt und tropfenweise mit einer Lösung von 1,2 g (0,010 8 Mol) 4-Amino-1-methyl-piperidin in 10 ml Acetonitril versetzt. Man erhitzte 2 Stunden unter Rückfluß, saugte heiß ab und dampfte das Filtrat im Vakuum ein. Der Rückstand wurde säulenchromatographisch gereinigt (Kieselgel, Fließmittel : Methylenchlorid + Cyclohexan + Methanol + Ammoniak = 102 + 23 + 23 + 3). Die gewünschte Fraktion wurde im Vakuum eingedampft, der Rückstand wurde aus Acetonitril umkristallisiert. Nadeln vom F. 206-208 °C. Die Ausbeute betrug 50 % der Theorie.

Entsprechend erhielt man :

aus 4-Chlorcarbonyl-4,9-dihydro-10H-thieno [3,4-b] [1,5] benzodiazepin-10-on und 1 Methyl-4-methylamino-piperidin das 4,9-Dihydro-4-[[N-methyl-N-(1-methyl-4-piperidinyl) amino]-carbonyl]-10H-thieno [3,4-b] [1,5] benzodiazepin-10-on ;

aus 4,9-Dihydro-4-chlorcarbonyl-10H-thieno [3,4-b] [1,5] benzodiazepin-10-on und endo-3-Amino-8-methyl-8-azabicyclo [3,2,17-octan das endo-4,9-Dihydro-4-[[(8-methyl-8-azabicyclo [3,2,1]-oct-3-yl) amino] carbonyl]-10H-thieno [3,4-b] [1,5] benzodiazepin-10-on vom F. 203-205 °C (Acetonitril) ;

aus 4-Chlorcarbonyl-4,9-dihydro-1-methyl-10H-thieno [3,4-b] [1,5] benzodiazepin-10-on und 4-Amino-1-methyl-piperidin das 4,9-Dihydro-1-methyl-4-[[1-methyl-4-piperidinyl) amino]-carbonyl]-10H-thieno [3,4-b] [1,5] benzodiazepin-10-on vom F. 240,5-241 °C (aus Essigsäureethylester) ;

aus 4-Chlorcarbonyl-4,9-dihydro-3-methyl-10H-thieno [3,4-b] [1,5] benzodiazepin-10-on und 4-Amino-1-methyl-piperidin das 4,9-Dihydro-3-methyl-4-[[1-methyl-4-piperidinyl) amino]-carbonyl]-10H-thieno [3,4-b] [1,5] benzodiazepin-10-on ;

aus 4-Chlorcarbonyl-4,9-dihydro-1,3-dimethyl-10H-thieno [3,4-b] [1,5] benzodiazepin-10-on und 4-Amino-1-methyl-piperidin das 4,9-Dihydro-1,3-dimethyl-4-[[(1-methyl-4-piperidinyl) amino]-carbonyl]-10H-thieno [3,4-b] [1,5] benzodiazepin-10-on vom F. 136-140 °C (aus Acetonitril) ;

aus 3-Chlor-4-chlorcarbonyl-4,9-dihydro-10H-thieno [3,4-b] [1,5] benzodiazepin-10-on und 4-Amino-1-methyl-piperidin das 3-Chlor-4,9-dihydro-4-[[(1-methyl-4-piperidinyl) amino]-carbonyl]-10H-thieno [3,4-b] [1,5] benzodiazepin-10-on ;

aus 4-Chlorcarbonyl-4,9-dihydro-10H-thieno [3,4-b] [1,5] benzodiazepin-10-on und endo-8-Methyl-3-methylamino-8-azabicyclo [3,2,1] octan das endo-4,9-Dihydro-4-[[N-methyl-N-(8-methyl-8-azabicyclo [3,2,1] oct-3-yl) amino] carbonyl]-10H-thieno [3,4-b] [1,5] benzodiazepin-10-on vom F. 259-260 °C (Ethanol).

aus 4-Chlorcarbonyl-4,9-dihydro-10H-thieno [3,4-b] [1,5] benzodiazepin-10-on und 1-Amino-4-methyl-piperazin das 4,9-Dihydro-4-[[(4-methyl-1-piperazinyl) amino] carbonyl]-10H-thieno [3,4-b] [1,5] benzo-diazepin-10-on vom F. 240-241 °C (2-Propanol) ;

aus 4-Chlorcarbonyl-4,9-dihydro-1-methyl-10H-thieno [3,4-b] [1,5] benzodiazepin-10-on und 1-Amino-4-methylpiperazin das 4,9-Dihydro-1-methyl-4-[[(4-methyl-1-piperazinyl) amino]-carbonyl]-10H-thieno [3,4-b] [1,5] benzodiazepin-10-on ;

aus 4-Chlorcarbonyl-4,9-dihydro-3-methyl-10H-thieno [3,4-b] [1,5] benzodiazepin-10-on und 1-Amino-4-methyl-piperazin das 4,9-Dihydro-3-methyl-4-[[(4-methyl-1-piperazinyl) amino]-carbonyl]-10H-thieno [3,4-b] [1,5] benzodiazepin-10-on ;

aus 4-Chlorcarbonyl-4,9-dihydro-1,3-dimethyl-10H-thieno-[3,4-b] [1,5] benzodiazepin-10-on und 1-Amino-4-methyl-piperazin das 4,9-Dihydro-1,3-dimethyl-4-[[(4-methyl-1-piperazinyl) amino]-carbonyl]-10H-thieno [3,4-b] [1,5] benzodiazepin-10-on ;

aus 3-Chlor-4-chlorcarbonyl-4,9-dihydro-10H-thieno [3,4-b] [1,5] benzodiazepin-10-on und 1-Amino-4-methyl-piperazin das 3-Chlor-4,9-dihydro-4-[[(4-methyl-1-piperazinyl) amino] carbonyl]-10H-thieno [2,4-b] [1,5] benzodiazepin-10-on ;

aus 4-Chlorcarbonyl-4,9-dihydro-10H-thieno [3,4-b] [1,5] benzodiazepin-10-on und exo-3-Amino-8-methyl-8-azabicyclo [3,2,1] octan das exo-4,9-Dihydro-4-[[(8-methyl-8-azabicyclo [3,2,1]-oct-3-yl) amino] carbonyl]-10H-thieno [3,4-b] [1,5]benzodiazepin-10-on vom F. 235-237 °C (Acetonitril) ;

aus 4-Chlorcarbonyl-4,9-dihydro-1-methyl-10H-thieno [3,4-b] [1,5] benzodiazepin-10-on und endo-3-Amino-8-methyl-8-azabicyclo [3,2,1] octan das endo-4,9-Dihydro-1-methyl-4-[[(8-methyl-8-azabicyclo [3,2,1] oct-3-yl) amino] carbonyl]-10H-thieno-[3,4-b] [1,5] benzodiazepin-10-on ;

Aus 4-Chlorcarbonyl-4,9-dihydro-3-methyl-10H-thieno [3,4-b] [1,5] benzodiazepin-10-on und endo-3-Amino-8-methyl-8-azabicyclo [3,2,1] octan das endo-4,9-Dihydro-3-methyl-4-[[(8-methyl-8-azabicyclo [3,2,1] oct-3-yl) amino] carbonyl]-10H-thieno [3,4-b] [1,5] benzodiazepin-10-on ;

aus 4-Chlorcarbonyl-4,9-dihydro-1,3-dimethyl-10H-thieno [3,4-b] [1,5] benzodiazepin-10-on und endo-3-Amino-8-methyl-8-azabicyclo [3,2,1] octan das endo-4,9-Dihydro-1,3-dimethyl-4[[(8-methyl-8-aza-bicyclo [3,2,1] oct-3-yl) amino] carbonyl]-10H-thieno [3,4-b] [1,5] benzodiazepin-10-on ;

aus 3-Chlor-4-chlorcarbonyl-4,9-dihydro-10H-thieno [3,4-b] [1,5] benzodiazepin-10-on und endo-3-Amino-8-methyl-8-azabicyclo [3,2,1] octan das endo-3-Chlor-4,9-dihydro-4-[[(8-methyl-8-azabicyclo [3,2,1] oct-3-yl) amino] carbonyl]-10H-thieno [3,4-b] [1,5] benzodiazepin-10-on vom F. 158-161 °C (Acetonitril) ;

aus 3-Chlor-4-chlorcarbonyl-4,9-dihydro-10H-thieno [3,4-b] [1,5] benzodiazepin-10-on und exo-3-Amino-8-methyl-8-azabicyclo-[3,2,1] octan das exo-3-Chlor-4,9-dihydro-4-[[(8-methyl-8-azabicyclo [3,2,1] oct-3-yl) amino] carbonyl]-10H-thieno [3,4-b] [1,5] benzodiazepin-10-on vom F. 224-225 °C (aus Acetonitril).

## Beispiel 3

4,9-Dihydro-4-[[(1-methyl-4-piperidinyl) oxy] carbonyl]-10H-thieno [3,4-b] [1,5] benzodiazepin-10-on

Aus 4-Chlorcarbonyl-4,9-dihydro-10H-thieno [3,4-b] [1,5] benzodiazepin-10-on und 1-Methyl-4-piperi-dinol nach der im Beispiel 2 beschriebenen Methode. F. 189-190 °C (aus Acetonitril). Ausbeute : 55 % der Theorie.

Die erhaltene Base wurde in Ethylacetat gelöst und mit einer Lösung von Chlorwasserstoff in Dioxan versetzt. Das ausgefallene Hydrochlorid wurde aus einem Gemisch von Ethanol : Diethylether = 1 : 1 umkristallisiert. F. : 220-222 °C (Z.).

Entsprechend erhielt man :

aus 4-Chlorcarbonyl-4,9-dihydro-1-methyl-10H-thieno [3,4-b] [1,5]-benzodiazepin-10-on und 1-Methyl-4-piperidinol das 4,9-Dihydro-1-methyl-4-[[(1-methyl-4-piperidinyl) oxy] carbonyl]-10H-thieno [3,4-b] [1,5] benzodiazepin-10-on vom F. 201-202 °C (Essigsäureethylester) ;

aus 4-Chlorcarbonyl-4,9-dihydro-3-methyl-10H-thieno [3,4-b] [1,5] benzodiazepin-10-on und 1-Methyl-4-piperidinol das 4,9-Dihydro-3-methyl-4-[[(1-methyl-4-piperidinyl) oxy] carbonyl]-10H-thieno [3,4-b] [1,5] benzodiazepin-10-on ;

aus 4-Chlorcarbonyl-4,9-dihydro-1,3-dimethyl-10H-thieno-[3,4-b] [1,5] benzodiazepin-10-on und 1-Methyl-4-piperidinol das 4,9-Dihydro-1,3-dimethyl-4-[[(1-methyl-4-piperidinyl) oxy]-carbonyl-10H-thieno [3,4-b] [1,5] benzodiazepin-10-on vom F. 198-200 °C (Essigsäureethylester) ;

aus 3-Chlor-4-chlorcarbonyl-4,9-dihydro-10H-thieno [3,4-b] [1,5] benzodiazepin-10-on und 1-Methyl-4-piperidinol das 3-Chlor-4,9-dihydro-4-[[(1-methyl-4-piperidinyl) oxy] carbonyl]-10H-thieno [3,4-b] [1,5] benzodiazepin-10-on ;

aus 4-Chlorcarbonyl-4,9-dihydro-10H-thieno [3,4-b] [1,5] benzodiazepin-10-on und 1-Hydroxy-4-methyl-piperazin das 4,9-Dihydro-4-[[(4-methyl-1-piperazinyl) oxy] carbonyl]-10H-thieno-[3,4-b] [1,5] benzodiazepin-10-on ;

aus 4-Chlorcarbonyl-4,9-dihydro-1-methyl-10H-thieno [3,4-b] [1,5] benzodiazepin-10-on und 1-Hydroxy-4-methyl-piperazin das 4,9-Dihydro-1-methyl-4-[[(4-methyl-1-piperazinyl) oxy] carbonyl]-10H-thieno [3,4b] [1,5] benzodiazepin-10-on ;

aus 4-Chlorcarbonyl-4,9-dihydro-10H-thieno [3,4-b] [1,5] benzodiazepin-10-on und endo-8-Methyl-8-azabicyclo [3,2,1] octan-3-ol das endo-4,9-Dihydro-4-[[(8-methyl-8-azabicyclo [3,2,1] oct-3-yl) oxy] carbonyl]-10H-thieno [3,4-b] [1,5] benzodiazepin-10-on ;

aus 4-Chlorcarbonyl-4,9-dihydro-1-methyl-10H-thieno [3,4-b] [1,5] benzodiazepin-10-on und endo-8-Methyl-8-azabicyclo [3,2,1] octan-3-ol das endo-4,9-Dihydro-1-methyl-4-[[(8-methyl-8-azabicyclo [3,2,1]oct-3-yl) oxy] carbonyl]-10H-thieno [3,4-b] [1,5] benzodiazepin-10-on ;

aus 4-Chlorcarbonyl-4,9-dihydro-3-methyl-10H-thieno [3,4-b] [1,5] benzodiazepin-10-on und endo-8-Methyl-8-azabicyclo [3,2,1] octan-3-ol das endo-4,9-Dihydro-3-methyl-4-[[(8-methyl-8-azabicyclo [3,2,1] oct-3-yl) oxy [carbonyl]-10H-thieno [3,4-b] [1,5]-benzodiazepin-10-on ;

aus 4-Chlorcarbonyl-4,9-dihydro-1,3-dimethyl-10H-thieno-[3,4-b] [1,5] benzodiazepin-10-on und endo-8-Methyl-8-azabicyclo [3,2,1] octan-3-ol das endo-4,9-Dihydro-1,3-dimethyl-4-[[(8-methyl-8-azabicyclo [3,2,1] oct-3-yl) oxy] carbonyl]-10H-thieno [3,4-b] [1,5] benzodiazepin-10-on ;

aus 3-Chlor-4-chlorcarbonyl-4,9-dihydro-10H-thieno [3,4-b] [1,5] benzodiazepin-10-on und endo-8-Methyl-8-azabicyclo [3,2,1] octan-3-ol das endo-3-Chlor-4,9-dihydro-4-[[(8-methyl-8-azabicyclo [3,2,1] oct-3-yl) oxy] carbonyl]-10H-thieno [3,4-b] [1,5] benzodiazepin-10-on ;

aus 4-Chlorcarbonyl-4,9-dihydro-10H-thieno [3,4-b] [1,5] benzodiazepin-10-on und exo-8-Methyl-8-azabicyclo [3,2,1] octan-3-ol das exo-4,9-Dihydro-4-[[(8-methyl-8-azabicyclo [3,2,1] oct-3-yl) oxy] carbonyl]-10H-thieno [3,4-b] [1,5] benzodiazepin-10-on.

Im folgenden wird die Herstellung pharmazeutischer Anwendungsformen anhand einiger Beispiele beschrieben ;

## Beispiel I

Tabletten mit 5 mg 4,9-Dihydro-4-[[(1-methyl-4-piperidinyl) oxy] carbonyl]-10H-thieno [3,4-b] [1,5] benzodiazepin-10-on-hydrochlorid

Zusammensetzung :
1 Tablette enthält :

| | |
|---|---|
| Wirkstoff | 5,0 mg |
| Milchzucker | 148,0 mg |
| Kartoffelstärke | 65,0 mg |
| Magnesiumstearat | 2,0 mg |
| | 220,0 mg |

Herstellungsverfahren :

Aus Kartoffelstärke wird durch Erwärmen ein 10 %iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45 °C getrocknet, nochmals durch obiges Sieb gerieben, mit Magnesiumstearat vermischt und zu Tabletten verpreßt.

Tablettengewicht : 220 mg
Stempel : 9 mm

## Beispiel II

Dragées mit 5 mg 4,9-Dihydro-4-[[(1-methyl-4-piperidinyl) oxy] carbonyl]-10H-thieno [3,4-b] [1,5] benzodiazepin-10-on-hydrochlorid

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht : 300 mg

## Beispiel III

Ampullen mit 1 mg 4,9-Dihydro-4-[[(1-methyl-4-piperidinyl) oxy] carbonyl]-10H-thieno [3,4-b] [1,5] benzodiazepin-10-on-hydrochlorid

Zusammensetzung :
1 Ampulle enthält :

| | |
|---|---|
| Wirkstoff | 1,0 mg |
| Natriumchlorid | 8,0 mg |
| Dest. Wasser ad | 1 ml |

Herstellungsverfahren :

Die Wirksubstanz und Natriumchlorid werden in dest. Wasser gelöst und anschließend auf das gegebene Volumen aufgefüllt. Die Lösung wird sterilfiltriert und in 1ml-Ampullen abgefüllt. Sterilisation : 20 Minuten bei 120 °C.

Beispiel IV

Suppositorien mit 5 mg 4,9-Dihydro-4-[[(1-methyl-4-piperidinyl) oxy] carbonyl]-10H-thieno [3,4-b] [1,5] benzodiazepin-10-on-hydrochlorid
Zusammensetzung :
1 Zäpfchen enthält :

| | |
|---|---|
| Wirkstoff | 5,0 mg |
| Zäpfchenmasse (z. B. Witepsol W 45®) | 1 695,0 mg |
| | 1 700,0 mg |

Herstellungsverfahren :

Die feinpulverisierte Wirksubstanz wird in der geschmolzenen und auf 40 °C abgekühlten Zäpfchenmasse suspendiert. Man gießt die Masse bei 37 °C in leicht vorgekühlte Zäpfchenformen aus. Zäpfchengewicht : 1,7 g

Beispiel V

Tropfen mit 4,9-Dihydro-4-[[(1-methyl-4-piperidinyl) oxy] carbonyl]-10H-thieno [3,4-b] [1,5] benzodiazepin-10-on-hydrochlorid enthaltend 0,5 g Wirkstoff/100 ml Lösung

Zusammensetzung :
100 ml Tropflösung enthalten :

| | |
|---|---|
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,5 g |
| Natriumcyclamat | 1,0 g |
| Glycerin | 15,0 g |
| Dest. Wasser ad | 100,0 ml |

Herstellungsverfahren :

Die Wirksubstanz und Natriumcyclamat werden in ca. 70 ml Wasser gelöst und Glycerin zugefügt. Man löst p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

**Patentansprüche**

1. Substituierte Thienobenzodiazepinone der allgemeinen Formel I

(Siehe Figur Seite 16 f.)

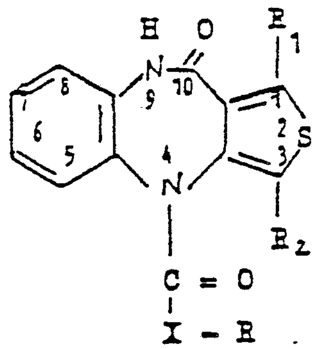

(I)

worin

$R_1$ ein Wasserstoffatom oder einen Alkylrest mit 1 bis 4 Kohlenstoffatomen,

$R_2$ ein Halogenatom, ein Wasserstoffatom oder einen Alkyl-rest mit 1 bis 4 Kohlenstoffatomen,

X ein Sauerstoffatom oder die —NH- oder —$NCH_3$-Gruppe und

R eine gegebenenfalls durch eine weitere Methylgruppe substituierte 1-Methyl-4-piperidinyl-, 4-Methyl-1-piperazinyl- oder eine 3α- oder 3β-Tropanylgruppe bedeuten, gegebenenfalls ihre geometrischen Isomeren und Enantiomeren und ihre physiologisch verträglichen Salze mit anorganischen oder organischen Säuren.

2. Substituierte Thienobenzodiazepinone der allgemeinen Formel I, in der

$R_1$ ein Wasserstoffatom oder die Methylgruppe,

$R_2$ ein Chlor- oder Wasserstoffatom oder die Methylgruppe,

X das Sauerstoffatom oder die —NH- oder —$NCH_3$-Gruppe und

R die 1-Methyl-4-piperidinyl-, 4-Methyl-1-piperazinyl-, endo- oder exo-8-Methyl-8-azabicyclo [3,2,1] oct-3-yl-gruppe (= 3α- oder 3β-Tropanylgruppe)

bedeuten, gegebenenfalls ihre geometrischen Isomeren und Enantiomeren und ihre pharmakologisch verträglichen Salze mit anorganischen oder organischen Säuren.

3. 4,9-Dihydro-4-[[(1-methyl-4-piperidinyl) oxy] carbonyl]-10H-thieno [3,4-b] [1,5] benzodiazepin-10-on und dessen pharmakologisch verträgliche Salze mit anorganischen oder organischen Säuren.

4. 4,9-Dihydro-4-[[(1-methyl-4-piperidinyl) amino] carbonyl]-10H-thieno [3,4-b] [1,5] benzodiazepin-10-on und dessen pharmakologisch verträgliche Salze mit anorganischen oder organischen Säuren.

5. 4,9-Dihydro-4-[[(4-methyl-1-piperazinyl) amino] carbonyl]-10H-thieno [3,4-b] [1,5] benzodiazepin-10-on und dessen pharmakologisch verträgliche Salze mit anorganischen oder organischen Säuren.

6. Arzneimittel enthaltend eine Substanz der allgemeinen Formel I gemäß den Ansprüchen 1 bis 5 neben den üblichen Träger- und/oder Hilfsstoffen.

7. Substituierte Thienobenzodiazepinone als Zwischenprodukte der allgemeinen Formel Ia

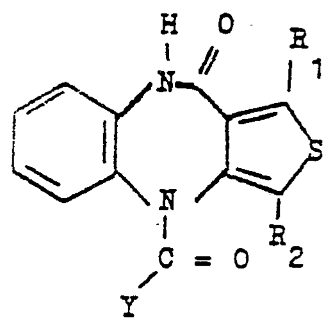

(Ia)

in der

$R_1$ ein Wasserstoffatom oder einen Alkylrest mit 1 bis 4 Kohlenstoffatomen,

$R_2$ ein Halogenatom, ein Wasserstoffatom oder einen Alkylrest mit 1 bis 4 Kohlenstoffatomen,

Y ein Halogenatom oder die Gruppe —$OR_3$

bedeuten, worin $R_3$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt.

8. Verfahren zur Herstellung von substituierten Thienobenzodiazepinonen der allgemeinen Formel I

(Siehe Figur Seite 17 f.)

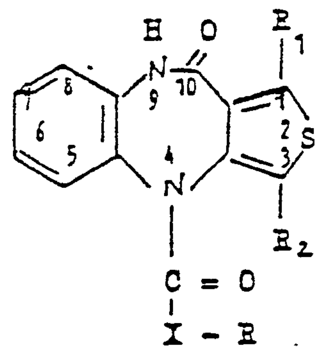

(I)

worin

$R_1$ ein Wasserstoffatom oder einen Alkylrest mit 1 bis 4 Kohlenstoffatomen,

$R_2$ ein Halogenatom, ein Wasserstoffatom oder einen Alkylrest mit 1 bis 4 Kohlenstoffatomen,

X ein Sauerstoffatom oder die —NH- oder —$NCH_3$-Gruppe und

R eine gegebenenfalls durch eine weitere Methylgruppe substituierte 1-Methyl-4-piperidinyl-, 4-Methyl-1-piperazinyl- oder eine 3α- oder 3β-Tropanylgruppe

bedeuten, und von ihren Salzen mit physiologisch verträglichen anorganischen oder organischen Säuren, dadurch gekennzeichnet, daß Thienobenzodiazepinone der allgemeinen Formel Ia

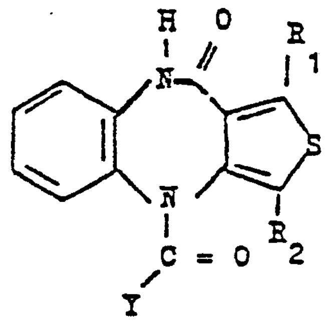

(Ia)

worin $R_1$ und $R_2$ wie oben definiert sind und Y ein Halogenatom oder eine Gruppe —$OR_3$ bedeutet, in der $R_3$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt,

a) mit Verbindungen der allgemeinen Formel II

$$H—X—R \qquad (II)$$

in der X und R die oben angegebenen Bedeutungen haben, ohne Lösungsmittel, gegebenenfalls auch in Gegenwart inerter Lösungsmittel bei Temperaturen zwischen 0 °C und dem Siedepunkt des Reaktionsgemisches, gegebenenfalls in Gegenwart eines Überschusses der Verbindungen der allgemeinen Formel II oder einer anorganischen oder organischen Base, oder

b) mit Metallverbindungen der allgemeinen Formel IIa

$$M—X—R \qquad (IIa)$$

in der M ein Alkalimetallatom oder 1 Äquivalent eines Erdalkalimetallatoms bedeutet, unter denselben Bedingungen umgesetzt oder

c) Verbindungen der allgemeinen Formel III

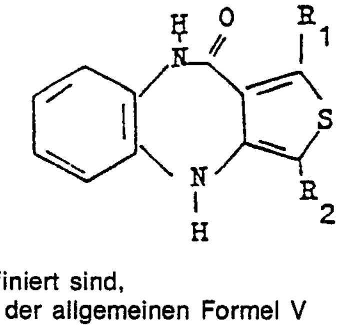

(III)

in der $R_1$ und $R_2$ wie oben definiert sind,

mit Chlorkohlensäurederivaten der allgemeinen Formel V

$$Cl - \underset{\underset{O}{\|}}{C} - X - R \qquad (V)$$

oder mit Isocyanaten der allgemeinen Formel Va

$$O = C = N—R \qquad (Va)$$

in welchen X und R wie oben definiert sind, in einem inerten Lösungsmittel umgesetzt werden, gegebenenfalls eine Auftrennung in die Isomeren nach an sich üblichen Methoden erfolgt und gewünschtenfalls so erhaltene Verbindungen der allgemeinen Formel I anschließend mittels physiologisch verträglicher anorganischer oder organischer Säuren in ihre Salze übergeführt werden.

9. Verfahren zur Herstellung von Verbindungen der allgemeinen Formel Ia

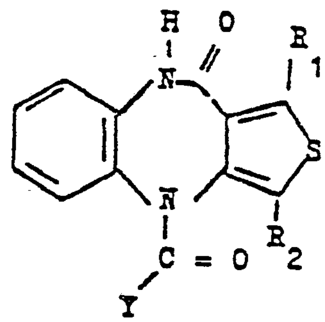

(Ia)

in der

$R_1$ ein Wasserstoffatom oder einen Alkylrest mit 1 bis 4 Kohlenstoffatomen,

$R_2$ ein Halogenatom, ein Wasserstoffatom oder einen Alkylrest mit 1 bis 4 Kohlenstoffatomen,

Y ein Halogenatom oder die Gruppe $—OR_3$

bedeuten, worin $R_3$ einen gegebenenfalls halogensubstituierten Alkylrest mit 1 bis 5 Kohlenstoffatomen, einen gegebenenfalls durch Halogen oder Nitro substituierten Phenylrest oder einen Aralkylrest mit 7 bis 15 Kohlenstoffatomen darstellt, dadurch gekennzeichnet, daß Thienobenzodiazepinone der allgemeinen Formel III

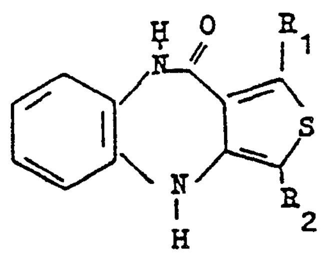

(III)

in der $R_1$ und $R_2$ wie oben definiert sind, mit Halogenkohlensäurederivaten der allgemeinen Formel IV

$$Hal - \underset{\underset{O}{\|}}{C} - Y \qquad (IV)$$

in der Hal ein Chlor- oder Bromatom bedeutet und Y die oben angegebenen Bedeutüngen hat, in inerten organischen Lösungsmitteln und in Gegenwart von tertiären organischen Basen bei Temperaturen zwischen 30 und 100 °C umgesetzt werden.

10. Verfahren zur Trennung optisch aktiver Isomeren der allgemeinen Formel I, dadurch gekennzeichnet, daß Verbindungen der allgemeinen Formel I mit optisch aktiven Säuren in äquimolaren Mengen in einem Lösungsmittel umgesetzt werden und die erhaltenen kristallinen, optisch aktiven Salze unter Ausnutzung ihrer verschiedenen Löslichkeiten voneinander getrennt werden.

**Claims**

1. Substituted thienobenzodiazepinones of general formula I

(See figure page 19)

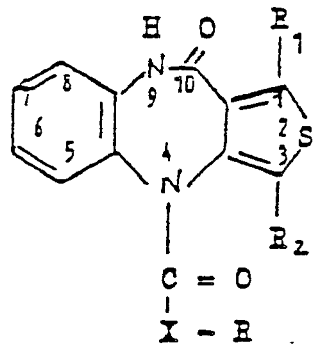

(I)

wherein

$R_1$ represents a hydrogen atom or an alkyl group with 1 to 4 carbon atoms,

$R_2$ represents a halogen atom, a hydrogen atom or an alkyl group with 1 to 4 carbon atoms,

X represents an oxygen atom or the —NH- or —$NCH_3$-group and

R represents a 1-methyl-4-piperidinyl, 4-methyl-1-piperazinyl or a 3α- or 3β-tropanyl group, each group being optionally substituted by another methyl group, and possibly the geometric isomers and enantiomers thereof and the physiologically acceptable salts thereof with inorganic or organic acids.

2. Substituted thienobenzodiazepinones of general formula I, wherein

$R_1$ represents a hydrogen atom or the methyl group,

$R_2$ represents a chlorine or hydrogen atom or the methyl group,

X represents the oxygen atom or the —NH- or —$NCH_3$-group, and

R represents the 1-methyl-4-piperidinyl, 4-methyl-1-piperazinyl, endo- or exo-8-methyl-8-azabicyclo [3,2,1] oct-3-yl group (= 3α- or 3β-tropanyl group), and optionally the geometric isomers and enantiomers thereof and the pharmacologically acceptable salts thereof with inorganic or organic acids.

3. 4,9-Dihydro-4-[[(1-methyl-4-piperidinyl) oxyl] carbonyl]-10H-thieno [3,4-b] [1,5] benzodiazepin-10-one and the pharmacologically acceptable salts thereof with inorganic or organic acids.

4. 4,9-Dihydro-4-[[(1-methyl-4-piperidinyl) amino] carbonyl]-10H-thieno [3,4-b] [1,5] benzodiazepin-10-one and the pharmacologically acceptable salts thereof with inorganic or organic acids.

5. 4,9-Dihydro-4-[[(4-methyl-1-piperazinyl) amino] carbonyl]-10H-thieno [3,4-b] [1,5] benzodiazepin-10-one and the pharmacologically acceptable salts thereof with inorganic or organic acids.

6. Pharmaceutical compositions containing a substance of general formula I as claimed in claims 1 to 5 together with the conventional carriers and/or excipients.

7. Substituted thienobenzodiazepinones as intermediate products of general formula Ia,

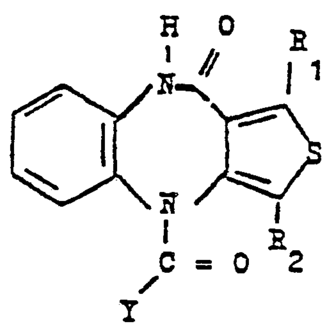

(Ia)

wherein

$R_1$ represents a hydrogen atom or an alkyl group with 1 to 4 carbon atoms,

$R_2$ represents a halogen atom, a hydrogen atom or an alkyl group with 1 to 4 carbon atoms,

Y represents a halogen atom or the group —$OR_3$, wherein $R_3$ represents an optionally halogen-substituted alkyl group with 1 to 5 carbon atoms, a phenyl group optionally substituted by halogen or nitro or an aralkyl group with 7 to 15 carbon atoms.

8. Process for the preparation of substituted thienobenzodiazepinones of general formula I

(See figure page 20)

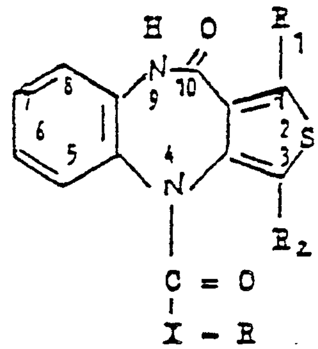

(I)

wherein

$R_1$ represents a hydrogen atom or an alkyl group with 1 to 4 carbon atoms,

$R_2$ represents a halogen atom, a hydrogen atom or an alkyl group with 1 to 4 carbon atoms,

X represents an oxygen atom or the —NH- or —$NCH_3$-group and

R represents a 1-methyl-4-piperidinyl, 4-methyl-1-piperazinyl or a 3α- or 3β-tropanyl group, each group being optionally substituted by another methyl group, and of the salts thereof with physiologically acceptable inorganic or organic acids, characterised in that thienobenzodiazepinones of general formula Ia

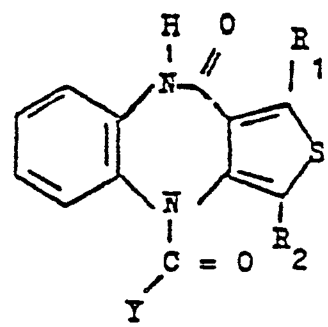

(Ia)

wherein $R_1$ and $R_2$ are as hereinbefore defined and Y represents a halogen atom or a group —$OR_3$ wherein $R_3$ represents an optionally halogen-substituted alkyl group with 1 to 5 carbon atoms, a phenyl group optionally substituted by halogen or nitro or an aralkyl group with 7 to 15 carbon atoms,

a) are reacted with compounds of general formula II

H—X—R (II)

wherein X and R are as hereinbefore defined, without a solvent, possibly also in the presence of inert solvents at temperatures between 0 °C and the boiling point of the reaction mixture, optionally in the presence of an excess of the compounds of general formula II or of an inorganic or organic base, or

b) are reacted with metal compounds of general formula IIa

M—X—R (IIa)

wherein M represents an alkali metal atom or 1 equivalent of an alkaline earth metal atom, under the same conditions, or

c) compounds of general formula III

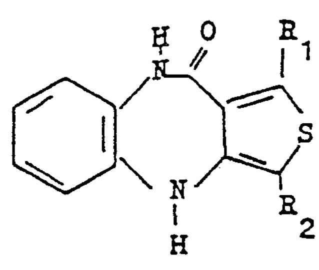

(III)

wherein $R_1$ and $R_2$ are as hereinbefore defined, are reacted with chlorocarbonic acid derivatives of general formula V

Cl - C - X - R
‖
O
(V)

or with isocyanates of general formula Va

$$O = C = N—R \qquad (Va)$$

wherein X and R are as hereinbefore defined, in an inert solvent, and optionally the compounds are resolved into the isomers according to conventional methods and, if desired, compounds of general formula I thus obtained are subsequently converted into their salts with physiologically acceptable inorganic or organic acids.

9. Process for the preparation of compounds of general formula Ia

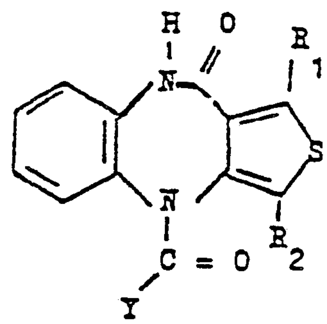

(Ia)

wherein

$R_1$ represents a hydrogen atom or an alkyl group with 1 to 4 carbon atoms,

$R_2$ represents a halogen atom, a hydrogen atom or an alkyl group with 1 to 4 carbon atoms,

Y represents a halogen atom or the group $—OR_3$, wherein $R_3$ represents an optionally halogen-substituted alkyl group with 1 to 5 carbon atoms, a phenyl group optionally substituted by halogen or nitro or an aralkyl group with 7 to 15 carbon atoms, characterised in that thienobenzodiazepinones of general formula III

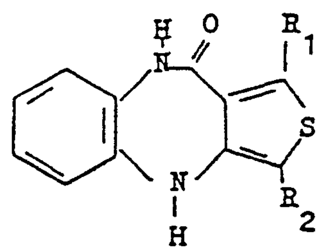

(III)

wherein $R_1$ and $R_2$ are as hereinbefore defined, are reacted with halocarbonic acid derivatives of general formula IV

Hal - C - Y
‖
O
(IV)

wherein Hal represents a chlorine or bromine atom and Y is as hereinbefore defined, in inert organic solvents and in the presence of tertiary organic bases at temperatures of between 30 and 100 °C.

10. Process for separating optically active isomers of general formula I, characterised in that compounds of general formula I are reacted with optically active acids in equimolar quantities in a solvent and the crystalline, optically active salts obtained are separated from one another, by making use of their different solubilities.

**Revendications**

1. Thiénobenzodiazépinones substituées de formule générale I :

(Voir figure page 22)

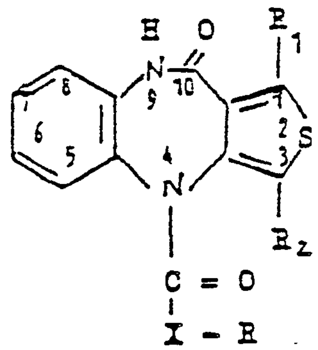

(I)

dans laquelle :

$R_1$ représente un atome d'hydrogène ou un radical alcoyle avec 1 à 4 atomes de carbone,

$R_2$ représente un atome d'halogène, un atome d'hydrogène ou un radical alcoyle avec 1 à 4 atomes de carbone,

X représente un atome d'oxygène ou le groupe —NH— ou —$NCH_3$— et

R représente un groupe 1-méthyl-4-pipéridinyle, 4-méthyl-1-pipérazinyle, éventuellement substitué par un autre groupe méthyle, ou représente un groupe 3α- ou 3β-tropanyle, éventuellement leurs isomères et énantiomères géométriques et leurs sels physiologiquement supportables avec des acides minéraux ou organiques.

2. Thiénobenzodiazépinones substituées de formule générale I, dans laquelle :

$R_1$ représente un atome d'hydrogène ou le groupe méthyle,

$R_2$ représente un atome de chlore ou d'hydrogène ou le groupe méthyle,

X représente l'atome d'oxygène ou le groupe —NH— ou —$NCH_3$— et

R représente le groupe 1-méthyl-4-pipéridinyle, 4-méthyl-1-pipérazinyle, endo- ou exo-8-méthyl-8-azabicyclo [3,2,1]-oct-3-yle (= 3α- ou 3β-tropanyle), éventuellement leurs isomères et énantiomères géométriques et leurs sels pharmacologiquement supportables avec des acides minéraux ou organiques.

3. La 4,9-dihydro-4-[[(1-méthyl-4-pipéridinyl) oxy] carbonyl]-10H-thiéno [3,4-b] [1,5] benzodiazépine-10-one et ses sels pharmacologiquement supportables avec des acides minéraux ou organiques.

4. La 4,9-dihydro-4-[[(1-méthyl-4-pipéridinyl) amino] carbonyl]-10H-thiéno [3,4-b] [1,5] benzodiazépine-10-one et ses sels pharmacologiquement supportables avec des acides minéraux ou organiques.

5. La 4,9-dihydro-4-[[(4-méthyl-1-pipérazinyl) amino] carbonyl]-10H-thiéno [3,4-b] [1,5] benzodiazépine-10-one et ses sels pharmacologiquement supportables avec des acides minéraux ou organiques.

6. Médicament contenant une substance de formule générale I selon les revendications 1 à 5 conjointement aux excipients et/ou adjuvants usuels.

7. Thiénobenzodiazépinones substituées en tant que produits intermédiaires de formule générale Ia :

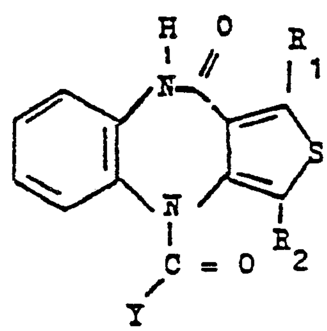

(Ia)

dans laquelle :

$R_1$ représente un atome d'hydrogène ou un radical alcoyle avec 1 à 4 atomes de carbone,

$R_2$ représente un atome d'halogène, un atome d'hydrogène ou un radical alcoyle avec 1 à 4 atomes de carbone,

Y représente un atome d'halogène ou le groupe —$OR_3$, dans lequel $R_3$ représente un radical alcoyle avec 1 à 5 atomes de carbone, éventuellement halogéno-substitué, un radical phényle éventuellement substitué par halogène ou nitro, ou un radical aralcoyle avec 7 à 15 atomes de carbone.

8. Procédé pour la préparation de thiénobenzodiazépinones substituées de formule générale I :

(Voir figure page 23)

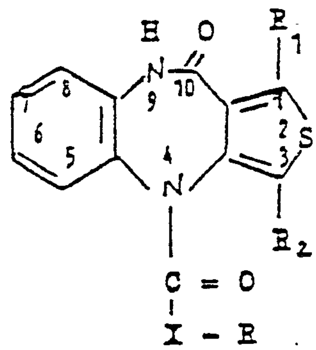

(I)

dans laquelle :

$R_1$ représente un atome d'hydrogène, un radical alcoyle avec 1 à 4 atomes de carbone,

$R_2$ représente un atome d'halogène, un atome d'hydrogène ou un radical alcoyle avec 1 à 4 atomes de carbone,

X représente un atome d'oxygène ou le groupe —NH— ou —$NCH_3$— et

R représente un groupe 1-méthyl-4-pipéridinyle, 4-méthyl-1-pipérazinyle, éventuellement substitué par un autre groupe méthyle, ou représente un groupe $3\alpha$- ou $3\beta$-tropanyle, et de leurs sels avec des acides minéraux ou organiques physiologiquement supportables, caractérisé en ce que l'on fait réagir des thiénobenzodiazépinones de formule générale Ia :

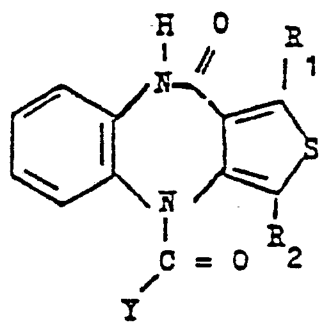

(Ia)

dans laquelle $R_1$ et $R_2$ sont définis comme plus haut, et Y représente un atome d'halogène ou un groupe —$OR_3$, dans lequel $R_3$ représente un radical alcoyle avec 1 à 5 atomes de carbone, éventuellement halogéno-substitué, un radical phényle éventuellement substitué par halogène ou nitro ou un radical aralcoyle avec 7 à 15 atomes de carbone,

a) avec des composés de formule générale II :

H—X—R (II)

dans laquelle X et R ont les significations indiquées plus haut, sans solvant, éventuellement aussi en présence de solvants inertes à des températures entre 0 °C et le point d'ébullition du mélange réactionnel, éventuellement en présence d'un excès des composés de formule générale II ou d'une base minérale ou organique, ou

b) avec des composés métalliques de formule générale IIa :

M—X—R (IIa)

dans laquelle M représente un atome de métal alcalin ou un équivalent d'un atome de métal alcalino-terreux, dans les mêmes conditions ou

c) on fait réagir des composés de formule générale III :

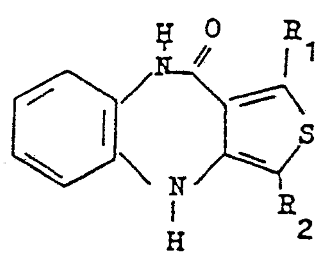

(III)

dans laquelle $R_1$ et $R_2$ sont définis comme plus haut, avec des dérivés d'acide chlorocarbonique de formule générale V :

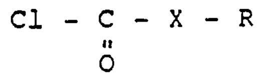

(V)

ou avec des isocyanates de formule générale Va :

$$O = C = N—R \qquad (Va)$$

dans lesquelles X et R sont définis comme plus haut, dans un solvant inerte, éventuellement a lieu une séparation en les isomères selon des méthodes usuelles en soi et si on le désire, des composés ainsi obtenus de formule générale I sont ensuite transformés, au moyen d'acides minéraux ou organiques physiologiquement supportables, en leurs sels.

9. Procédé pour la préparation de composés de formule générale Ia :

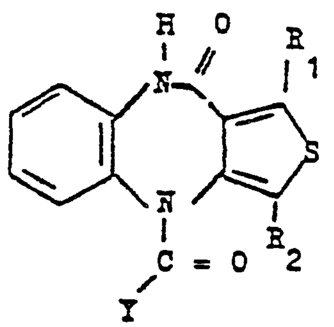

(Ia)

dans laquelle :

$R_1$ représente un atome d'hydrogène ou un radical alcoyle avec 1 à 4 atomes de carbone,

$R_2$ représente un atome d'halogène, un atome d'hydrogène ou un radical alcoyle avec 1 à 4 atomes de carbone,

Y représente un atome d'halogène ou le groupe $—OR_3$, dans lequel $R_3$ représente un radical alcoyle avec 1 à 5 atomes de carbone, éventuellement halogéno-substitué, un radical phényle éventuellement substitué par halogène ou nitro ou un radical aralcoyle avec 7 à 15 atomes de carbone, caractérisé en ce qu'on fait réagir des thiénobenzodiazépinones de formule générale III :

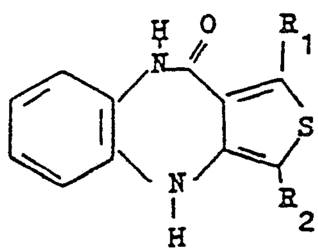

(III)

dans laquelle $R_1$ et $R_2$ sont définis comme plus haut, avec des dérivés d'acides halogénocarboniques de formule générale IV :

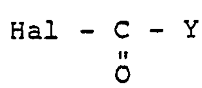

(IV)

dans laquelle Hal représente un atome de chlore ou de brome et Y a les significations indiquées plus haut, dans des solvants organiques inertes, et en présence de bases organiques tertiaires à des températures entre 30 et 100 °C.

10. Procédé pour la séparation d'isomères optiquement actifs de formule générale I, caractérisé en ce que l'on fait réagir des composés de formule générale I avec des acides optiquement actifs en quantités équimolaires dans un solvant et en ce qu'on sépare les uns des autres les sels optiquement actifs, cristallins, obtenus en utilisant leurs solubilités différentes.